# EUROPÄISCHE PATENTANMELDUNG

(11) **EP 3 926 636 A2**
(43) Veröffentlichungstag der Anmeldung: **22.12.2021**
(21) Anmeldenummer: 21172796.1
(22) Anmeldetag: 07.05.2021
(51) Int. Cl.: G16B 40/10, G16B 25/20

(54) **VERFAHREN ZUM ERKENNEN EINER AMPLIFIKATIONSPHASE IN EINER AMPLIFIKATION**

(30) Priorität: 18.06.2020 DE 102020116178
(71) Anmelder: Analytik Jena GmbH, 07745 Jena (DE)
(72) Erfinder: Möller, Eva, 07751 Jena (DE)
(74) Vertreter: Andres, Angelika Maria

(57) **Zusammenfassung**

Die Erfindung bezieht sich auf ein Verfahren zum Erkennen, ob eine Amplifikationsphase in einer Amplifikation, in welcher mindestens eine Nukleinsäure-Sequenz in einer definierten Anzahl von Amplifikationszyklen vermehrt wird, stattgefunden hat, wobei nach jedem Amplifikationszyklus die Intensität eines Signals der mindestens einen Nukleinsäure-Sequenz ermittelt und die ermittelte Intensität als Messpunkt in Abhängigkeit des Amplifikationszyklus dargestellt wird. Das Verfahren weist u.a. folgende Schritte auf: Berechnen einer ersten Regressionsgeraden (R1) von einem ersten Messpunkt, welcher nach einem ersten Amplifikationszyklus aufgenommen wurde, bis zum bestimmten Startpunkt der Amplifikationsphase (2); Berechnen einer zweiten Regressionsgerade (R2) über alle Messpunkte (MP) (3); Vergleichen der ersten Regressionsgerade (R1) mit der zweiten Regressionsgerade (R2) hinsichtlich der Unterschiedlichkeit von erster und zweiter Regressionsgerade (R1, R2) mittels eines ersten statistischen Tests, wobei ein erster applikationsspezifischer p-Wert (p1) ermittelt wird (4). Je nachdem, ob der erste applikationsspezifische p-Wert (p1) unterhalb oder oberhalb eines definierten Grenzwerts liegt, wird erkannt, dass eine Amplifikationsphase stattgefunden hat oder nicht (5,6).

## Beschreibung

Die Erfindung betrifft ein Verfahren zum Erkennen, ob eine Amplifikationsphase in einer Amplifikation, in welcher mindestens eine Nukleinsäure-Sequenz in einer definierten Anzahl von Amplifikationszyklen vermehrt wird, stattgefunden hat, wobei nach jedem Amplifikationszyklus die Intensität eines Signals der mindestens einen Nukleinsäure-Sequenz ermittelt und die ermittelte Intensität als Messpunkt in Abhängigkeit des Amplifikationszyklus dargestellt wird.

Eine Amplifikation ist eine Vermehrung von mindestens einer Nukleinsäure-Sequenz, also beispielsweise einer DNA-Sequenz. Die Vervielfältigung von Nukleinsäure-Sequenzen kann ein natürlich auftretender Prozess innerhalb der Genetik sein. Daneben werden Nukleinsäure-Sequenzen in der Molekularbiologie in vitro gezielt vermehrt, um beispielsweise Gene zu klonieren oder Krankheiten zu erkennen. Neben verschiedenen isothermalen Methoden zur Amplifikation ist die Polymerase-Kettenreaktion (polymerase chain reaction, PCR) die wohl bedeutendste Methode der Amplifikation.

In einer PCR wird eine Nukleinsäure-Sequenz, in der Regel ein kurzer Abschnitt der DNA, mittels eines Enzyms in einer definierten Anzahl von Amplifikationszyklen vermehrt. Das eingesetzte Enzym ist typischerweise eine sogenannte DNA-Polymerase. Nach jedem Amplifikationszyklus liegt die Nukleinsäure-Sequenz in verdoppelter Anzahl vor, so dass die Anzahl der Nukleinsäure-Sequenzen mit der Anzahl der Amplifikationszyklen exponentiell ansteigt. Eine typische Anzahl von Amplifikationszyklen liegt bei etwa 20-40 Amplifikationszyklen.

Ist neben der reinen Vermehrung der Nukleinsäure-Sequenz auch eine quantitative Analyse der Amplifikation erwünscht, kann auf eine der zahlreichen Variationen der PCR zurückgegriffen werden, wie beispielsweise der Echtzeit-PCR (real-time PCR), welche auch quantitative PCR (qPCR) genannt wird. Hier wird vor Beginn der Amplifikation den Nukleinsäure-Sequenzen ein Marker zugesetzt, welcher spezifisch an die Nukleinsäure-Sequenzen bindet und der dort gebunden Fluoreszenz emittiert. Auf diese Weise lässt sich nach jedem Amplifikationszyklus in Echt-Zeit die Intensität der Fluoreszenz der an den Nukleinsäure-Sequenzen gebunden Markern bestimmen. Im Idealfall weist die bestimmte Intensität der Fluoreszenz, welche über die Amplifikationszyklen aufgetragen wird, ein sigmoides Verhalten auf. In den ersten Amplifikationszyklen wird zunächst lediglich ein Hintergrundsignal gemessen, bis die Nukleinsäure-Sequenzen ausreichend stark vermehrt sind. Mit der exponentiell steigenden Anzahl der Nukleinsäure-Sequenzen steigt die Intensität der Fluoreszenz stark an, bevor sie in ein Plateau mündet, in dem keine weitere Vermehrung der Nukleinsäure-Sequenzen mehr stattfindet. Da die gemessene Intensität der Fluoreszenz der gebundenen Marker somit proportional zu der Anzahl an Nukleinsäure-Sequenzen ist, können verschiedene Modelle zur Quantifizierung der Amplifikation herangezogen werden.

Der sogenannte Schwellenzyklus (threshold cycle, Ct) gibt beispielsweise denjenigen Amplifikationszyklus an, in welchem die Intensität der Fluoreszenz der Marker erstmals signifikant über das (Fluoreszenz-)Hintergrundsignal ansteigt. Ein niedriger Cₜ-Wert bedeutet also, dass zu einem frühen Zeitpunkt eine große Anzahl von Nukleinsäure-Sequenzen vorliegt. Typischerweise wird keine einzelne Amplifikation durchgeführt, sondern eine ganze Reihe von Amplifikationen parallel durchgeführt und bevorzugt parallel ausgewertet. Für die Bestimmung des Cₜ-Werts bedeutet dies, dass für alle durchgeführten Amplifikationen eine gemeinsame Schwelle gefunden werden muss, anhand welcher der Cₜ-Wert bestimmt wird.

Für die Bestimmung des sogenannten Quantifizierungszyklus (quantification cycle, C_{q}) wird den während der Amplifikation bestimmten Messpunkten eine sigmoidale Modellfunktion angepasst, anhand welcher die Phase des exponentiellen Anstiegs der Fluoreszenz ermittelt wird. Der C_{q}-Wert kann als Alternative zum Cₜ-Wert bestimmt werden. Obwohl die Herangehensweise der Bestimmung der beiden Werte unterschiedlich ist, sollte im Idealfall derselbe amplification cycle bzw. Amplifikationszyklus als C_{q}-Wert und Cₜ-Wert erhalten werden. Wird eine Reihe von Amplifikationen durchgeführt, so sollten der C_{q}-Wert und der Cₜ-Wert für eine jeweilige Amplifikation idealerweise übereinstimmen, wobei innerhalb der Reihe von Amplifikationen für verschiedene Amplifikationen häufig verschiedene Werte des C_{q}-Werts und Cₜ-Werts erhalten werden.

Die Bestimmung des Schwellenzyklus (Cₜ) als auch des Quantifizierungszyklus (C_{q}) erweist sich dann als besonders schwierig, wenn die Amplifikationsphase nur schwach ausgeprägt und damit schwer zu identifizieren ist. Besonders in diesem Fall kommt es häufig zu Abweichungen des Quantifizierungszyklus (C_{q}) von dem Schwellenzyklus (Cₜ).

Die US 10 176 293 B2 gibt eine Methode an aus einer Amplifikation einen Quantifizierungsparameter systematisch zu bestimmen.

In der Patentschrift EP 1 472 518 B1 ist eine Methode zur Auswertung einer qPCR beschrieben, wobei eine Vielzahl von Amplifikationen parallel ausgewertet werden soll. Dabei wird eine Region eines exponentiellen Intensitätsanstiegs mit einer unteren und einer oberen Grenze sowie eine Hintergrundgerade bestimmt. Zusätzlich wird über alle Amplifikationen hinweg ein Grenzwert bestimmt, welcher zwischen der unteren und oberen Grenze des Intensitätsanstiegs liegt. Dieser Grenzwert gibt einen Amplifikationszyklus an, der als Schwellenzyklus bezeichnet wird, und der dazu dient, die einzelnen Amplifikationen quantitativ miteinander zu vergleichen.

Die CA 02736243 A1 beschreibt eine weitere Methode zur Auswertung einer PCR. In einem Schritt wird überprüft, ob eine Amplifikationsphase vorliegt. So werden beispielsweise zu kurze Messreihen oder Artefakte aussortiert, die nur scheinbar eine Amplifikation darstellen.

In der EP 1 647 910 A1 ist offenbart, dass die Intensität der Fluoreszenz mit einer Regressionsfunktion beschrieben wird, welche eine lineare und eine nichtlineare Funktion, insbesondere eine Sigmoidfunktion, enthält. Der Koeffizient der Sigmoidfunktion wird anschließend einem T-Test unterzogen, um zu überprüfen, mit welcher Wahrscheinlichkeit er ungleich Null ist, also wie wahrscheinlich die Intensität der Fluoreszenz einen nicht-linearen Anstieg aufweist. Messungen ohne eine Amplifikationsphase werden erst nachträglich aussortiert. Die Regressionsfunktion enthält zudem vier Parameter, wodurch die Regression an Unsicherheit gewinnt.

Die zu lösende Aufgabe der Erfindung ist es, ein Verfahren bereitzustellen, welches auf einfache Weise erkennt, ob eine Amplifikationsphase in einer Amplifikation stattgefunden hat.

Die Aufgabe wird erfindungsgemäß gelöst durch ein Verfahren zum Erkennen, ob eine Amplifikationsphase in einer Amplifikation, in welcher mindestens eine Nukleinsäure-Sequenz in einer definierten Anzahl von Amplifikationszyklen vermehrt wird, stattgefunden hat, wobei nach jedem Amplifikationszyklus die Intensität eines Signals der mindestens einen Nukleinsäure-Sequenz ermittelt und die ermittelte Intensität als Messpunkt in Abhängigkeit des Amplifikationszyklus dargestellt wird, wobei das Verfahren mindestens folgende Schritte aufweist:
- Bestimmen eines Startpunkts der Amplifikationsphase anhand der ermittelten Messpunkte, wobei der Startpunkt den Übergang zwischen einem im Wesentlichen konstanten Hintergrundsignal und einem Beginn der Amplifikationsphase markiert,
- Berechnen einer ersten Regressionsgerade von einem ersten Messpunkt, welcher nach einem ersten Amplifikationszyklus aufgenommen wurde, bis zum bestimmten Startpunkt der Amplifikationsphase,
- Berechnen einer zweiten Regressionsgerade über alle Messpunkte,
- Vergleichen der ersten Regressionsgerade mit der zweiten Regressionsgerade hinsichtlich der Unterschiedlichkeit von erster Regressionsgerade und zweiter Regressionsgerade mittels eines ersten

statistischen Tests, wobei ein erster applikationsspezifischer p-Wert ermittelt wird,
- für den Fall, dass der ermittelte erste applikationsspezifische p-Wert oberhalb eines definierten Grenzwerts liegt:
   ∘ Erkennen, dass keine Amplifikationsphase stattgefunden hat,
   ∘ Abbrechen einer weiteren Auswertung,
- für den Fall, dass der ermittelte erste applikationsspezifische p-Wert unterhalb eines definierten Grenzwerts liegt:
   ∘ Erkennen, dass eine Amplifikationsphase stattgefunden hat,
   ∘ Durchführen einer weiteren Auswertung.

Das erfindungsgemäße Verfahren beinhaltet zunächst die Bestimmung des Startpunkts der Amplifikationsphase, wobei der bestimmte Startpunkt je nach verwendeter Methode unter Umständen unterschiedlich sein kann. Es versteht sich von selbst, dass der Fachmann Artefakte und andere Messpunkte, die offensichtlich fehlerbehaftet sind, im Rahmen des erfindungsgemäßen Verfahrens nicht betrachtet. Im Anschluss werden die erste und die zweite Regressionsgerade berechnet. Dies erfolgt in bekannter weise durch eine lineare Regression anhand der jeweiligen Messpunkte, wobei ein Maximum der jeweiligen Anpassungsgüte der ersten und zweiten Regressionsgeraden erreicht werden soll. Die erste Regressionsgerade wird zwischen dem ersten Messpunkt und dem bestimmten Startpunkt der Amplifikationsphase berechnet, so dass die erste Regressionsgerade eine Hintergrundintensität der Fluoreszenz beschreibt. Die zweite Regressionsgerade wird über alle Messpunkte berechnet und schließt damit eine Amplifikationsphase mit ein, falls eine Amplifikationsphase stattgefunden hat.

Im nächsten Schritt werden die erste und die zweite Regressionsgerade hinsichtlich ihrer Unterschiedlichkeit mittels des ersten statistischen Tests verglichen, beispielsweise anhand der jeweiligen Steigung der ersten und zweiten Regressionsgeraden. Dazu werden insbesondere die Messpunkte zwischen dem ersten Messpunkt und dem bestimmten Startpunkt der Amplifikationsphase herangezogen. In dem Fall, dass eine Amplifikationsphase stattgefunden hat, sollten sich die erste und die zweite Regressionsgerade voneinander unterscheiden, da die Amplifikationsphase im Gegensatz zu dem Fluoreszenz-Hintergrund einen nicht-linearen Anstieg aufweist.

In der Regel wird eher erwartet, dass in einer Amplifikation eine Amplifikationsphase stattgefunden hat. In der Statistik wird als Nullhypothese das Ergebnis definiert, das dem erwarteten Ergebnis gegenübersteht, also in diesem Fall, dass keine Amplifikationsphase stattgefunden hat. Übertragen auf den Vergleich der ersten und zweiten Regressionsgeraden würde man eher erwarten, dass sich beide voneinander unterscheiden. Die entsprechende Nullhypothese des ersten statistischen Tests lautet also, dass sich erste und zweite Regressionsgerade ähnlich sind.

Der erste statistische Test gibt einen ersten applikationsspezifischen p-Wert aus, anhand dessen relativ zu einem definierten Grenzwert erkannt wird, ob eine Amplifikationsphase stattgefunden hat oder nicht. Ein niedriger erster applikationsspezifischer p-Wert bedeutet, dass die erste und zweite Regressionsgerade hinreichend unterschiedlich sind, so dass auf eine Amplifikationsphase geschlossen werden kann. Ein erhöhter erster applikationsspezifischer p-Wert signalisiert, dass die erste und zweite Regressionsgerade ähnlich zueinander sind und damit keine Amplifikationsphase stattgefunden hat.

Das erfindungsgemäße Verfahren ermöglicht es also auf einfache Weise zu erkennen, ob eine Amplifikationsphase stattgefunden hat oder nicht. Wird erkannt, dass keine Amplifikationsphase stattgefunden hat, so wird die weitere Auswertung abgebrochen und keine weitere Zeit und Energie in die Auswertung gesteckt. Wird dagegen erkannt, dass eine Amplifikationsphase stattgefunden hat, kann eine weitere Auswertung der Messpunkte erfolgen, um quantitative und/oder qualitative Informationen über die Amplifikationsphase zu erhalten. Innerhalb diagnostischer Anwendungen kann typischerweise erwartet werden, dass 60-80 % der ausgewerteten Amplifikationen eine Amplifikationsphase enthalten. Damit kann mittels des erfindungsgemäßen Verfahrens bei bis zu 40% der durchgeführten Amplifikationen vorab erkannt werden, dass keine Amplifikationsphase stattgefunden hat und die weitere Auswertung eingespart werden.

Darüber hinaus bietet das erfindungsgemäße Verfahren den Vorteil, dass der Fachmann eine Sicherheit erhält, dass die weitere Auswertung bei einer erkannten Amplifikationsphase sinnvoll ist, wodurch die Vertrauenswürdigkeit der nachfolgenden Ergebnisse der weiteren Auswertung steigt. Dies gilt insbesondere für solche Amplifikationen, in denen die Amplifikationsphase schwer zu identifizieren ist, da die Messpunkte beispielsweise stark schwanken.

Der Grenzwert kann sich beispielsweise an üblichen Grenzwerten für p-Werte orientieren. Typischerweise wird eine Reihe von Amplifikationen parallel durchgeführt. Zusätzlich werden innerhalb dieser Reihe Positiv- und Negativkontrollen vermessen, die zur Überprüfung eines systematischen Fehlers in der gemessenen Reihe der Amplifikationen dienen. Für eine Positivkontrolle wird ein kleiner erster applikationsspezifischer p-Wert erwartet, für eine Negativkontrolle hingegen ein hoher erster applikationsspezifischer p-Wert. Wenn also der erste applikationsspezifische p-Wert einer Positivkontrolle den Grenzwert überschreitet, dann können alle weiteren Amplifikationen derselben Reihe als fehlerbehaftet angesehen werden und werden nicht weiter ausgewertet. Umgekehrt gilt die Reihe an Amplifikationen ebenso als fehlerbehaftet, wenn für eine Negativkontrolle innerhalb der Reihe ein erster applikationsspezifischer p-Wert unterhalb des Grenzwertes bestimmt wird.

Alternativ kann der Grenzwert anhand der ersten applikationsspezifischen p-Werte von mindestens einer Positivkontrolle und/oder mindestens einer Negativkontrolle innerhalb einer Reihe von Amplifikationen ermittelt werden. So wird erkannt, dass eine Amplifikationsphase stattgefunden hat, wenn ein erster applikationsspezifischer p-Wert einer Amplifikation kleiner ist als der niedrigste erste applikationsspezifische p-Wert innerhalb derjenigen ersten applikationsspezifischen p-Werte, die für die mindestens eine Negativkontrolle bestimmt wurden, oder wenn ein erster applikationsspezifischer p-Wert kleiner ist als der größte erste applikationsspezifische p-Wert innerhalb derjenigen ersten applikationsspezifischen p-Werte, die für die mindestens eine Positivkontrolle bestimmt wurden.

In einer möglichen Weiterentwicklung weist das Verfahren folgende Schritte auf:
- Anpassen einer ersten Modellfunktion, welche zumindest eine Sigmoidfunktion enthält, an alle Messpunkte in einem ersten Schritt,
- Anpassen einer quadratischen Modellfunktion an alle Messpunkte in einem zweiten Schritt,
- Vergleichen der ersten Modellfunktion mit der quadratischen Modellfunktion hinsichtlich der Unterschiedlichkeit der ersten Modellfunktion und der quadratischen Modellfunktion mittels eines zweiten statistischen Tests, wobei ein zweiter applikationsspezifischer p-Wert ermittelt wird,
- für den Fall, dass der ermittelte zweite applikationsspezifische p-Wert oberhalb eines definierten Grenzwerts liegt:
   ∘ Erkennen, dass keine Amplifikationsphase stattgefunden hat,
- für den Fall, dass der ermittelte zweite applikationsspezifische p-Wert unterhalb eines definierten Grenzwerts liegt:
   ∘ Erkennen, dass eine Amplifikationsphase stattgefunden hat.

In der weiteren Auswertung, also im Falle einer erkannten Amplifikationsphase, wird untersucht, ob der erwartete sigmoidale Verlauf der Amplifikationsphase tatsächlich vorliegt. Hierzu wird die erste Modellfunktion, welche den erwarteten sigmoidalen Verlauf der Amplifikationsphase wiedergeben soll, mit der quadratischen Modellfunktion verglichen, welche keinen stetigen exponentiellen Anstieg aufweist. Die Erwartung ist also, dass ein Unterschied zwischen der quadratischen und der ersten Modellfunktion gefunden wird. Die Nullhypothese lautet daher, dass sich die quadratische Modellfunktion und die erste Modellfunktion ähnlich sind. Mittels eines zweiten statistischen Tests wird diese Nullhypothese anschließend überprüft, wobei ein zweiter applikationsspezifischer p-Wert erhalten wird. Je nach Lage des zweiten applikationsspezifischen p-Werts relativ zu einem definierten Grenzwert wird erkannt, dass eine Amplifikationsphase stattgefunden hat oder nicht.

Während der erste statistische Test überprüft, ob in der Amplifikation ein nicht-linearer Anstieg enthalten ist und dadurch abschätzt, ob eine Amplifikationsphase stattgefunden hat, überprüft der zweite statistische Test den exponentiellen Anstieg der Amplifikationsphase. Mittels des ersten applikationsspezifischen p-Werts kann somit eine Vorauswahl derjenigen Amplifikationen getroffen werden, in denen wahrscheinlich eine Amplifikationsphase stattgefunden hat. Der zweite applikationsspezifische p-Wert liefert eine Aussage über das Vorhandensein eines exponentiellen Anstiegs in der Amplifikation. Somit werden der erste und der zweite applikationsspezifische p-Werte erhalten und können beide genutzt werden, um eine Amplifikationsphase festzustellen.

In einer Weiterbildung des erfindungsgemäßen Verfahrens werden die erste Regressionsgerade und die zweite Regressionsgerade hinsichtlich der Unterschiedlichkeit von erster Regressionsgerade und zweiter Regressionsgerade anhand der Varianzen der Residuen der Messpunkte zur ersten Regressionsgerade sowie zur zweiten Regressionsgerade mittels des ersten statistischen Tests verglichen. Es werden also zunächst die Residuen der Messpunkte zur ersten und zur zweiten Regressionsgerade gebildet, sowie im Anschluss die Varianzen der Residuen der Messpunkte. Der erste statistische Test analysiert die Varianzen der Residuen der Messpunkte zur ersten und zur zweiten Regressionsgerade nach Unterschieden.

Vorteilhafterweise wird als erster statistischer Test ein F-Test verwendet. Ein F-Test dient zur generellen Überprüfung von Unterschieden zwischen zwei statistischen Populationen anhand der Varianzen der beiden statistischen Populationen. Beispielsweise überprüft der F-Test die Varianzen der Residuen der Messpunkte zur ersten Regressionsgerade sowie zur zweiten Regressionsgerade auf Unterschiede.

In einer weiteren Weiterbildung des erfindungsgemäßen Verfahrens werden die erste Modellfunktion und die quadratische Modellfunktion hinsichtlich der Unterschiedlichkeit der ersten Modellfunktion und der quadratischen Modellfunktion anhand der Varianzen der Residuen der Messpunkte zur ersten Modellfunktion sowie zur quadratischen Funktion mittels des zweiten statistischen Tests verglichen. Der zweite statistische Test analysiert, analog zum ersten statistischen Test, die Varianzen der Residuen der Messpunkte zur ersten und zur quadratischen Modellfunktion nach Unterschieden

Bevorzugterweise wird als zweiter statistischer Test ein F-Test verwendet. Beispielsweise überprüft der F-Test die Varianzen der Residuen der Messpunkte zur ersten Modellfunktion sowie zur quadratischen Modellfunktion auf Unterschiede.

Eine mögliche Weiterbildung sieht vor, dass die erste Modellfunktion als eine Summe aus einer Sigmoidfunktion und einer linearen Funktion gebildet wird. In dem Fall gibt die Sigmoidfunktion den Verlauf der Amplifikationsphase wieder, während die lineare Funktion eine Hintergrundintensität beschreibt. Da die erste Regressionsgerade die Hintergrundintensität wiedergibt, kann die erste Regressionsgerade vorzugsweise als lineare Funktion für die erste Modellfunktion eingesetzt werden.

In einer weiteren Weiterbildung wird als Grenzwert des ermittelten ersten und/oder zweiten applikationsspezifischen p-Werts bevorzugt ein p-Wert von 0,05 oder von 0,01 oder zwischen 0,01 und 0,05 definiert. Der Grenzwert orientiert sich typischerweise an den allgemein üblichen Schwellen oder Schwellwerten für p-Werte.

In einer möglichen weiteren Weiterbildung wird die erste Regressionsgerade als Hintergrundgerade von der Gesamtheit der Messpunkte subtrahiert. Typischerweise wird nach dem zweiten statistischen Test und bei Bestätigung des Vorliegens einer Amplifikationsphase die erste Regressionsgerade als Hintergrundgerade von allen Messpunkten abgezogen und so die Hintergrundintensität eliminiert. Dies erleichtert im Anschluss die Bestimmung eines Quantifizierungsparameters.

Vorteilhafterweise wird als Signalintensität der mindestens einen Nukleinsäure-Sequenz die Fluoreszenz oder Absorbanz der mindestens einen Nukleinsäure-Sequenz gemessen. Häufig weist die mindestens eine Nukleinsäure-Sequenz in einem relevanten Wellenlängenbereich keine oder kaum eine Absorbanz auf, die für die Auswertung einer Amplifikation benutzt werden könnte. Daher werden die Nukleinsäure-Sequenzen häufig mit Marker-Molekülen zusammengeführt, die sich an die Nukleinsäure-Sequenzen angliedern und eine Fluoreszenz aufweisen.

Bevorzugterweise wird als Technik der Amplifikation eine Polymerase-Kettenreaktion eingesetzt. Insbesondere wird eine Echtzeit-PCR oder qPCR eingesetzt.

In einer bevorzugten Weiterbildung wird der Wert des ermittelten ersten und/oder zweiten applikationsspezifischen p-Werts eingesetzt, um die Qualität mindestens eines Quantifizierungsparameters zu bestimmen, wobei der mindestens eine Quantifizierungsparameter ein Maß für die Konzentration der Nukleinsäure-Sequenzen ist und anhand der Intensität des Signals der Nukleinsäure-Sequenzen bestimmt wird. Der erste und/oder zweite applikationsspezifische p-Wert soll den mindestens einen Quantifizierungsparameter nicht ersetzen, sondern bietet ein Maß für die Verlässlichkeit und Vertrauenswürdigkeit des bestimmten Quantifizierungsparameters. Beispielsweise kann ein Quantifizierungsparameter, welcher mit einem niedrigen ersten applikationsspezifischen p-Wert einhergeht, als verlässlicher betrachtet werden, als ein Quantifizierungsparameter, der mit einem vergleichsweise hohen ersten applikationsspezifischen p-Wert einhergeht.

Vorteilhafterweise wird als mindestens einer Quantifizierungsparameter ein Schwellenzyklus (Ct-Wert) und/oder ein Quantifizierungszyklus (Cq-Wert) bestimmt. Insbesondere wenn der Ct-Wert vom Cq-Wert derselben Amplifikation abweicht, kann mittels des ersten und/oder zweiten applikationsspezifischen p-Werts überprüft werden, wie sicher eine Amplifikationsphase erkannt wurde und/oder wie gut der sigmoidale Verlauf der Amplifikationsphase angepasst werden konnte. Auch beim Vergleich verschiedener Amplifikationen mit unterschiedlichen Ct-Werten oder Cq-Werten kann der erste und/oder zweite applikationsspezifische p-Wert herangezogen werden, um eine Ursache der unterschiedlichen Ct-Werte oder Cq-Werte festzustellen. So können beispielsweise Cq-Werte, bei denen ein sehr niedriger erster und zweiter applikationsspezifischer p-Wert bestimmt wurde, als verlässlicher betrachtet werden als solche Cq-Werte, bei denen der erste und zweite applikationsspezifische p-Wert vergleichsweise höher ausfällt.

Eine mögliche Weiterbildung des erfindungsgemäßen Verfahrens sieht vor, dass der Startpunkt der Amplifikation mittels einer Methode eines gleitenden Fensters bestimmt wird, wobei aus allen Messpunkten eine definierte Anzahl von hintereinander ermittelten Messpunkten herausgenommen und als Fenster definiert wird, wobei ein spezifischer Parameter in jeweils der ersten und zweiten Hälfte des Fensters bestimmt wird, wobei anschließend das Fenster um einen Messpunkt verschoben und erneut ein spezifischer Parameter in jeweils der ersten und zweiten Hälfte des Fensters bestimmt wird, wobei das Fenster nach Bestimmung der spezifischen Parameter um jeweils einen Messpunkt verschoben wird, bis alle möglichen Fenster betrachtet und alle jeweiligen spezifischen Parameter bestimmt wurden, wobei als Startpunkt derjenige Messpunkt ausgewählt wird, bei dem der Unterschied der spezifischen Parameter aus der ersten und der zweiten Hälfte des Fensters am größten ist.

In einer möglichen Weiterbildung wird als spezifischer Parameter ein Mittelwert der Messpunkte oder ein Maximalwert der Messpunkte oder ein Anstieg einer Gerade, welche zwischen einem ersten Messpunkt des Fensters und einem mittleren Messpunkt des Fensters sowie zwischen dem mittleren Messpunkt des Fensters und einem letzten Messpunkt des Fensters gebildet wird, berechnet.

Das erfindungsgemäße Verfahren soll im Folgenden anhand der aufgeführten Figuren Fig. 1 - 5 näher erläutert werden. Es zeigt:
Fig. 1: ein Ausführungsbeispiel des erfindungsgemäßen Verfahrens.
Fig. 2a,b: jeweils einen Graph einer Amplifikation, in der eine Amplifikationsphase erkannt wird.
Fig. 3a,b: jeweils einen Graph einer Amplifikation, in der keine Amplifikationsphase erkannt wird.
Fig. 4a,b: jeweils einen Graph einer Amplifikation, in denen eine Amplifikationsphase nicht eindeutig erkannt wird.
Fig. 5: einen Graph einer Amplifikation, in dem die Methode der gleitenden Fenster zur Bestimmung des Startpunkts der Amplifikationsphase bestimmt eingesetzt wird.

Das erfindungsgemäße Verfahren kann für alle Amplifikationen, unabhängig von der verwendeten Technik oder dem verwendeten Apparat, verwendet werden. Das erfindungsgemäße Verfahren dient zum Erkennen, ob eine Amplifikationsphase in einer Amplifikation, in welcher mindestens eine Nukleinsäure-Sequenz in einer definierten Anzahl von Amplifikationszyklen vermehrt wird, stattgefunden hat. Dabei wird nach jedem Amplifikationszyklus die Intensität eines Signals der mindestens einen Nukleinsäure-Sequenz ermittelt und die ermittelte Intensität als Messpunkt in Abhängigkeit des Amplifikationszyklus dargestellt. Als Signal, dessen Intensität ermittelt wird, kann beispielsweise die Fluoreszenz oder Absorbanz der mindestens einen Nukleinsäure-Sequenz gemessen werden. Die Amplifikation kann beispielsweise mit der Technik der Polymerase-Kettenreaktion durchgeführt werden.

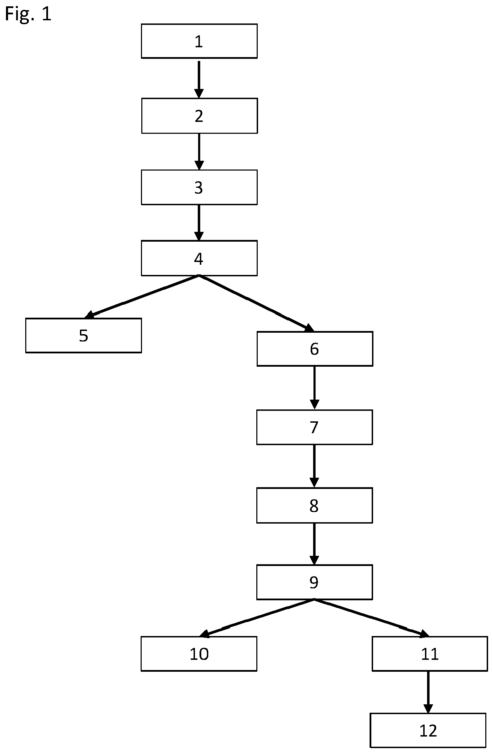

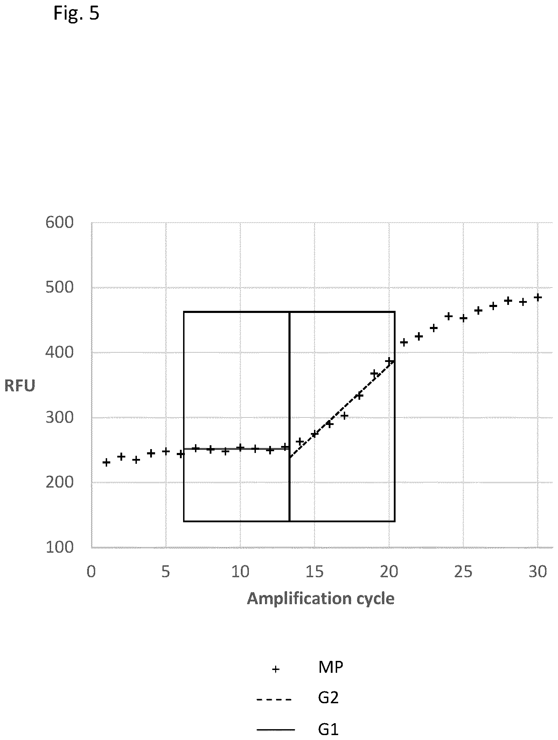

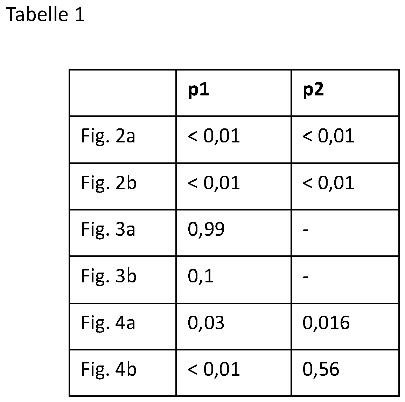

In Fig. 1 ist ein Ausführungsbeispiel des erfindungsgemäßen Verfahrens gezeigt. Im ersten Schritt 1 erfolgt das Bestimmen eines Startpunkts der Amplifikationsphase anhand der ermittelten Messpunkte MP, wobei der Startpunkt den Übergang zwischen einem im Wesentlichen konstanten Hintergrundsignal und einem Beginn der Amplifikationsphase markiert. Eine mögliche Methode zum Bestimmen des Startpunkts der Amplifikationsphase wird in Fig. 5 näher erläutert werden. Anschließend wird eine erste Regressionsgerade R1 von einem ersten Messpunkt MP, welcher nach einem ersten Amplifikationszyklus aufgenommen wurde, bis zum bestimmten Startpunkt SP der Amplifikationsphase in einem zweiten Schritt 2 berechnet. Im dritten Schritt 3 wird eine zweite Regressionsgerade R2 über alle Messpunkte MP berechnet.

Die erste Regressionsgerade R1 wird mit der zweiten Regressionsgeraden R2 hinsichtlich der Unterschiedlichkeit von erster Regressionsgerade R1 und zweiter Regressionsgerade R2 mittels eines ersten statistischen Tests verglichen (Schritt 4). Dabei werden insbesondere die Messpunkte MP zwischen dem ersten Messpunkt und dem Startpunkt SP der Amplifikationsphase herangezogen. Beispielsweise können mittels des ersten statistischen Tests die erste Regressionsgerade R1und die zweite Regressionsgerade R2 hinsichtlich der Unterschiedlichkeit von erster Regressionsgerade R1 und zweiter Regressionsgerade R2 anhand der Varianzen der Residuen der Messpunkte MP zur ersten Regressionsgerade sowie zur zweiten Regressionsgerade verglichen werden. Aus dem ersten statistischen Test, beispielsweise ein F-Test, wird ein erster applikationsspezifischer p-Wert ermittelt. Sofern der ermittelte erste applikationsspezifische p-Wert oberhalb eines definierten Grenzwerts liegt, wird in Schritt 5 erkannt, dass keine Amplifikationsphase stattgefunden hat und eine weitere Auswertung abgebrochen.

Sofern jedoch der ermittelte erste applikationsspezifische p-Werts unterhalb eines definierten Grenzwerts liegt, wird in Schritt 6 erkannt, dass eine Amplifikationsphase stattgefunden hat und die Auswertung der Amplifikation wird fortgesetzt. Anschließend wird in einem siebten Schritt 7 eine erste Modellfunktion F1 an alle Messpunkte MP angepasst. Die erste Modellfunktion F1 enthält eine Sigmoidfunktion und optional zusätzlich eine lineare Funktion. Als lineare Funktion kann insbesondere die erste Regressionsgerade R1 verwendet werden. In Schritt 8 wird eine quadratische Modellfunktion F2 an alle Messpunkte MP angepasst.

In Schritt 9 erfolgt, ähnlich wie in Schritt 4, ein Vergleich der ersten Modellfunktion F1 mit der quadratischen Modellfunktion F2 hinsichtlich ihrer Unterschiedlichkeit mittels eines zweiten quadratischen Tests, wobei ein zweiter applikationsspezifischer p-Wert ermittelt wird. Optional kann der Vergleich der ersten Modellfunktion F1 mit der quadratischen Modellfunktion F2 hinsichtlich ihrer Unterschiedlichkeit anhand der Varianzen der Residuen der Messpunkte MP zur ersten Modellfunktion sowie zur quadratischen Funktion mittels des zweiten statistischen Tests verglichen werden. Der zweite statistische Test kann beispielsweise ein F-Test sein.

Liegt der ermittelte zweite applikationsspezifische p-Wert oberhalb eines definierten Grenzwerts, so wird im Schritt 10 erkannt, dass keine Amplifikationsphase stattgefunden hat. Liegt der ermittelte zweite applikationsspezifische p-Werts hingegen unterhalb eines definierten Grenzwerts, wird in Schritt 11 erkannt, dass eine Amplifikationsphase stattgefunden hat. Der Grenzwert des ermittelten ersten und/oder zweiten applikationsspezifischen p-Werts wird beispielsweise als ein p-Wert von 0,05 oder von 0,01 oder zwischen 0,01 und 0,05 definiert. Der Grenzwert kann auch mithilfe von mindestens einer Positivkontrolle und/oder Negativkontrolle in einer Reihe von Amplifikationen festgelegt werden. Im Schritt 12 wird beispielsweise die Regressionsgerade R1 als Hintergrundgerade von der Amplifikationskurve für die anschließende optionale Bestimmung eines Quantifizierungsparameters subtrahiert. Der ermittelte erste und/oder zweite applikationsspezifische p-Wert kann optional dazu eingesetzt werden, die Qualität mindestens eines Quantifizierungsparameters zu bestimmen, wobei der mindestens eine Quantifizierungsparameter ein Maß für die Konzentration der Nukleinsäure-Sequenzen ist und anhand der Intensität des Signals der Nukleinsäure-Sequenzen bestimmt wird. Der mindestens eine Quantifizierungsparameter kann beispielsweise ein Schwellenzyklus (Ct-Wert) und/oder ein Quantifizierungszyklus (Cq-Wert) sein.

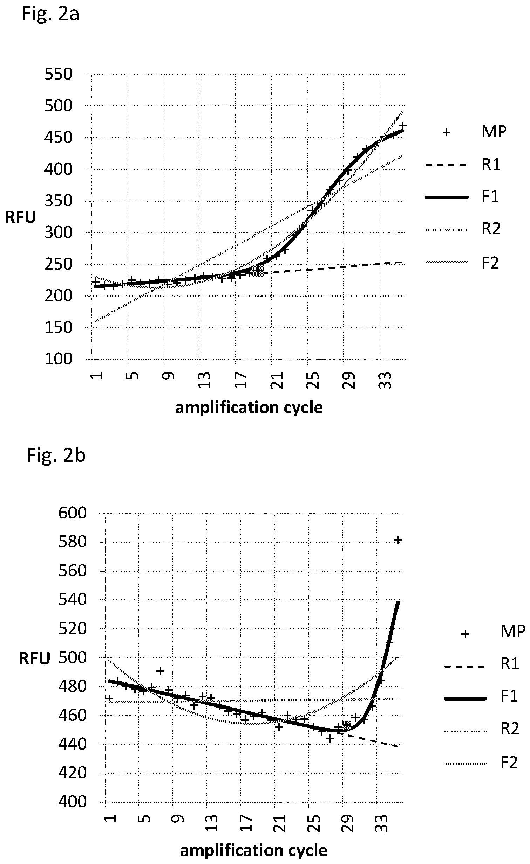

In den Figuren Fig. 2a - Fig. 4b sind beispielhafte Amplifikationen gezeigt, die mit dem erfindungsgemäßen Verfahren ausgewertet wurden. Dabei wurde die bestimmte (relative) Intensität der Fluoreszenz einer Nukleinsäure-Sequenz (relative fluorescence unit, RFU) als Messpunkte MP über dem Amplifikationszyklus oder amplification cycle aufgetragen. Es sind jeweils zu sehen: die erste Regressionsgerade R1, die zweite Regressionsgerade R2, die erste Modellfunktion F1 und die quadratische Modellfunktion F2. Die ermittelten ersten und zweiten applikationsspezifischen p-Werte p1,2 sind in der Tabelle 1 zusammengetragen. Der Startpunkt der Amplifikationsphase ist jeweils grau hinterlegt.

In den Figuren Fig. 2a,b wurde für die jeweilige Amplifikation als erster applikationsspezifischer p-Wert p1 ein Wert kleiner als 0,01 erhalten. Dieser sehr niedrige erste applikationsspezifische p-Wert p1 weist auf eine stattgefundene Amplifikationsphase hin. Daher wurde eine weitere Auswertung durchgeführt und die Messpunkte MP mit der ersten Modellfunktion F1 und der quadratischen Modellfunktion F2 angepasst. Aus dem zweiten statistischen Test wurde ein zweiter applikationsspezifischer p-Wert p2 erhalten, welcher für beiden Amplifikationen in den Figuren Fig. 2 a,b jeweils kleiner als 0,01 ist. Aufgrund der sehr niedrigen ersten und zweiten applikationsspezifischen p-Werte p1, p2 kann in Fig. 2 a,b mit hoher Sicherheit auf eine stattgefundene Amplifikationsphase geschlossen werden. Würde nun ein Quantifizierungsparameter anhand der Fig. 2 a,b bestimmt werden, so wäre der bestimmte Wert des Quantifizierungsparameters als sehr vertrauenswürdig anzusehen.

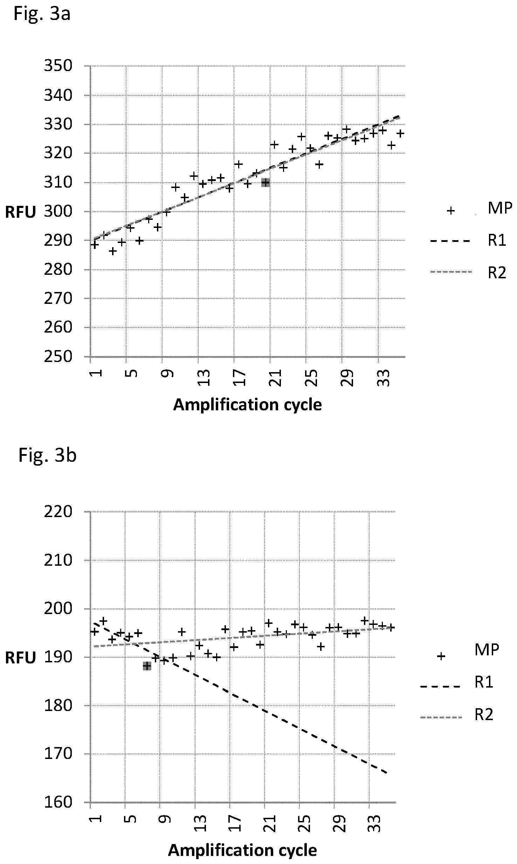

Die Figuren Fig. 3 a,b zeigen jeweils eine Amplifikation, bei der keine Amplifikationsphase stattgefunden hat. Die Durchführung des ersten statistischen Tests aus Basis der ersten und zweiten Regressionsgerade R1, R2 ergab in Fig. 3a einen ersten applikationsspezifischen p-Wert p1 von 0,99 und in Fig. 3b einen ersten applikationsspezifischen p-Wert p1 von 0,1. Diese beiden Werte für den ersten applikationsspezifischen p-Wert p1 entsprechen eher hohen Werten, weshalb postuliert wurde, dass keine Amplifikationsphase stattgefunden hat und keine weitere Auswertung durchgeführt wurde. Wäre in diesen beiden Beispielen ein Quantifizierungsparameter bestimmt worden, so wäre er durch den hohen ersten applikationsspezifischen p-Wert p1 mit einer großen Unsicherheit belegt bzw. kann als falsch betrachtet werden. Insbesondere für automatisierte Amplifikationsapparate, in denen eine hohe Zahl von Amplifikationen gleichzeitig durchgeführt wird, kann ein hoher erster applikationsspezifischer p-Wert p1 signalisieren, dass keine weitere Auswertung mehr möglich, womit der Zeitaufwand der weiteren Auswertung deutlich reduziert wird.

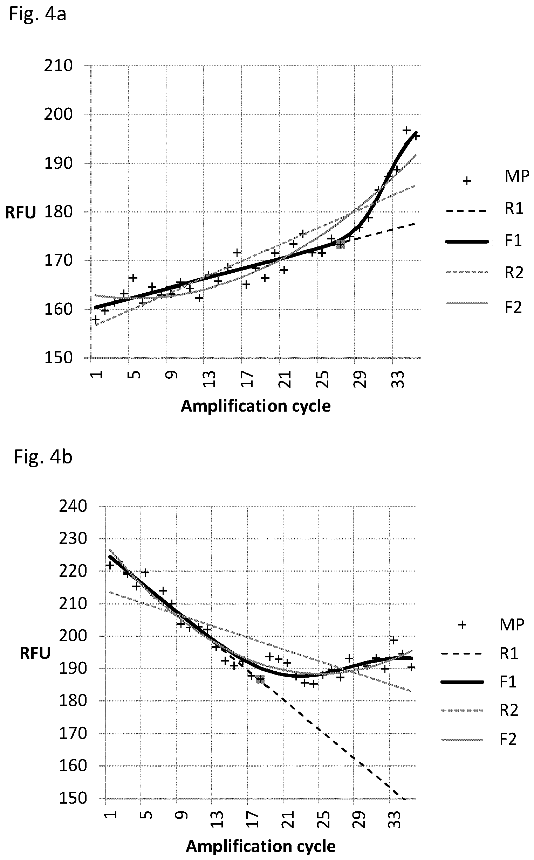

Es kann jedoch vorkommen, dass die Auswertung einer Amplifikation nicht so eindeutig ist, wie den bisher diskutierten Beispielen. Zwei solcher nicht-eindeutiger Beispiele sind in den Figuren Fig. 4 a,b gezeigt. In Fig. 4a wurde für die vorliegende Amplifikation als erster applikationsspezifischer p-Wert p1 ein Wert von 0,03 erhalten. Dieser Wert liegt in dem Bereich eines möglichen Grenzwerts von 0,01 bis 0,05, so dass je nach festgelegtem Grenzwert eine weitere Auswertung sinnvoll ist. Die weitere Auswertung ergab einen zweiten applikationsspezifischen p-Wert p2 mit einem Wert von 0,016, welcher auf eine signifikante Amplifikationsphase hinweist. Eine Betrachtung der Fig. 4a zeigt, dass tatsächlich eine Amplifikationsphase aufgetreten ist, jedoch erst in späteren Amplifikationszyklen, und mit einem geringen Anstieg.

In Fig. 4b wurde für die Amplifikation als erster applikationsspezifischer p-Wert p1 ein Wert kleiner als 0,01 erhalten, was deutlich auf das Vorliegen einer Amplifikationsphase hinweist. Die weitere Auswertung ergab jedoch einen zweiten applikationsspezifischen p-Wert p2 mit einem Wert von 0,56, was gegen das Auftreten einer Amplifikationsphase spricht. In diesem Beispiel wurde die Amplifikation durch einen externen Fehler gestört und die Amplifikationsphase ist als nicht vertrauenswürdig einzuschätzen, wie der zweite applikationsspezifische p-Wert p2 belegt. Insbesondere bei einem ersten applikationsspezifischen p-Wert, der nahe am Grenzwert liegt, kann es sinnvoll sein, den zweiten applikationsspezifischen p-Wert zu ermitteln, um anhand beider p-Werte eine Aussage über ein Auftreten einer Amplifikationsphase zu treffen. Sobald einer der beiden applikationsspezifischen p-Werte deutlich oberhalb des Grenzwerts liegt, kann dann von einem Fehlen der Amplifikationsphase ausgegangen werden.

In Fig. 5 wird anhand eines Graphs gezeigt, wie der Startpunkt der Amplifikationsphase mittels einer Methode eines gleitenden Fensters bestimmt wird. Dazu wird aus allen Messpunkten MP eine definierte Anzahl von hintereinander ermittelten Messpunkten MP herausgegriffen und als ein Fenster definiert. Das Fenster wird in zwei Hälften geteilt und ein spezifischer Parameter wird jeweils in der ersten und der zweiten Hälfte des Fensters bestimmt. In diesem Ausführungsbeispiel wurde in den beiden Hälften des 14 Messpunkte MP umfassenden Fensters jeweils eine Gerade G1, G2 durch die jeweils sieben Messpunkte MP in der jeweiligen Hälfte des Fensters gelegt, wobei der spezifische Parameter dem Anstieg der Geraden entspricht. Weitere Möglichkeiten für den spezifischen Parameter sind ein Mittelwert der Messpunkte MP oder ein Maximalwert der Messpunkte MP.

Nach der Bestimmung der spezifischen Parameter in jeder der zwei Hälften des Fensters wird das Fenster um einen Messpunkt MP verschoben und erneut die spezifischen Parameter in den beiden Hälften des Fensters bestimmt. Dieser Schritt wird sooft wiederholt, bis alle spezifischen Parameter in allen möglichen Fenstern bestimmt wurden. Der Startpunkt ist dann derjenige Messpunkt MP, an dem der Unterschied der spezifischen Parameter aus der ersten und der zweiten Hälfte des Fensters am größten ist, also beispielsweise der Anstieg der Geraden G1, G2 in der ersten und der zweiten Hälfte des Fenster die größte Differenz aufweist.

### Bezugszeichenliste

- MP: Messpunkt
- R1: erste Regressionsgerade
- R2: zweite Regressionsgerade
- F1: erste Modellfunktion
- F2: quadratische Modellfunktion
- p1: erster applikationsspezifischer p-Wert
- p2: zweiter applikationsspezifischer p-Wert
- G1: Gerade in der ersten Hälfte des Fensters
- G2: Gerade in der zweiten Hälfte des Fensters

## Patentansprüche

1. Verfahren zum Erkennen, ob eine Amplifikationsphase in einer Amplifikation, in welcher mindestens eine Nukleinsäure-Sequenz in einer definierten Anzahl von Amplifikationszyklen vermehrt wird, stattgefunden hat, wobei nach jedem Amplifikationszyklus die Intensität eines Signals der mindestens einen Nukleinsäure-Sequenz ermittelt und die ermittelte Intensität als Messpunkt in Abhängigkeit des Amplifikationszyklus dargestellt wird, wobei das Verfahren mindestens folgende Schritte aufweist:
- Bestimmen eines Startpunkts der Amplifikationsphase anhand der ermittelten Messpunkte (MP), wobei der Startpunkt den Übergang zwischen einem im Wesentlichen konstanten Hintergrundsignal und einem Beginn der Amplifikationsphase markiert (1),
- Berechnen einer ersten Regressionsgerade (R1) von einem ersten Messpunkt, welcher nach einem ersten Amplifikationszyklus aufgenommen wurde, bis zum bestimmten Startpunkt der Amplifikationsphase (2),
- Berechnen einer zweiten Regressionsgerade (R2) über alle Messpunkte (3),
- Vergleichen der ersten Regressionsgerade (R1) mit der zweiten Regressionsgerade (R2) hinsichtlich der Unterschiedlichkeit von erster Regressionsgerade (R1) und zweiter Regressionsgerade (R2) mittels eines ersten statistischen Tests, wobei ein erster applikationsspezifischer p-Wert (p1) ermittelt wird (4),
- für den Fall, dass der ermittelte erste applikationsspezifische p-Wert (p1) oberhalb eines definierten Grenzwerts liegt (5):
∘ Erkennen, dass keine Amplifikationsphase stattgefunden hat,
∘ Abbrechen einer weiteren Auswertung,
- für den Fall, dass der ermittelte erste applikationsspezifische p-Wert (p1) unterhalb eines definierten Grenzwerts liegt (6):
∘ Erkennen, dass eine Amplifikationsphase stattgefunden hat,
∘ Durchführen einer weiteren Auswertung.

2. Verfahren nach Anspruch 1,
wobei das Verfahren folgende Schritte aufweist:
- Anpassen einer ersten Modellfunktion (F1), welche zumindest eine Sigmoidfunktion enthält, an alle Messpunkte (MP) in einem ersten Schritt (7),
- Anpassen einer quadratischen Modellfunktion (F2) an alle Messpunkte (MP) in einem zweiten Schritt (8),
- Vergleichen der ersten Modellfunktion (F1) mit der quadratischen Modellfunktion (F2) hinsichtlich der Unterschiedlichkeit der ersten Modellfunktion (F1) und der quadratischen Modellfunktion (F2) mittels eines zweiten statistischen Tests, wobei ein zweiter applikationsspezifischer p-Wert (p2) ermittelt wird (9),
- für den Fall, dass der ermittelte zweite applikationsspezifische p-Wert (p2) oberhalb eines definierten Grenzwerts liegt (10):
∘ Erkennen, dass keine Amplifikationsphase stattgefunden hat,
- für den Fall, dass der ermittelte zweite applikationsspezifische p-Wert (p2) unterhalb eines definierten Grenzwerts liegt (11):
∘ Erkennen, dass eine Amplifikationsphase stattgefunden hat.

3. Verfahren nach mindestens einem der Ansprüche 1-2,
wobei die erste Regressionsgerade (R1) und die zweite Regressionsgerade (R2) hinsichtlich der Unterschiedlichkeit von erster Regressionsgerade (R1) und zweiter Regressionsgerade (R2) anhand der Varianzen der Residuen der Messpunkte (MP) zur ersten Regressionsgerade (R1) sowie zur zweiten Regressionsgerade (R2) mittels des ersten statistischen Tests verglichen werden.

4. Verfahren nach mindestens einem der Ansprüche 1-3,
wobei als erster statistischer Test ein F-Test verwendet wird.

5. Verfahren nach mindestens einem der Ansprüche 2-4,
wobei die erste Modellfunktion (F1) und die quadratische Modellfunktion (F2) hinsichtlich der Unterschiedlichkeit der ersten Modellfunktion (F1) und der quadratischen Modellfunktion (F2) anhand der Varianzen der Residuen der Messpunkte (MP) zur ersten Modellfunktion (F1) sowie zur quadratischen Funktion (F2) mittels des zweiten statistischen Tests verglichen werden.

6. Verfahren nach mindestens einem der Ansprüche 2-5,
wobei als zweiter statistischer Test ein F-Test verwendet wird.

7. Verfahren nach mindestens einem der Ansprüche 2-6,
wobei die erste Modellfunktion (F1) als eine Summe aus einer Sigmoidfunktion und einer linearen Funktion gebildet wird.

8. Verfahren nach mindestens einem der Ansprüche 1-7,
wobei als Grenzwert des ermittelten ersten und/oder zweiten applikationsspezifischen p-Werts (p1, p2) bevorzugt ein p-Wert von 0,05 oder von 0,01 oder zwischen 0,01 und 0,05 definiert wird.

9. Verfahren nach mindestens einem der Ansprüche 1-8,
wobei die erste Regressionsgerade (R1) als Hintergrundgerade von der Gesamtheit der Messpunkte subtrahiert wird (12).

10. Verfahren nach mindestens einem der Ansprüche 1-9,
wobei als Signalintensität der mindestens einen Nukleinsäure-Sequenz die Fluoreszenz oder Absorbanz der mindestens einen Nukleinsäure-Sequenz gemessen wird.

11. Verfahren nach mindestens einem der Ansprüche 1-10,
wobei als Technik der Amplifikation eine Polymerase-Kettenreaktion eingesetzt wird.

12. Verfahren nach mindestens einem der Ansprüche 1-11,
wobei der Wert des ermittelten ersten und/oder zweiten applikationsspezifischen p-Werts (p1, p2) eingesetzt wird, um die Qualität mindestens eines Quantifizierungsparameters zu bestimmen, wobei der mindestens eine Quantifizierungsparameter ein Maß für die Konzentration der Nukleinsäure-Sequenzen ist und anhand der Intensität des Signals der Nukleinsäure-Sequenzen bestimmt wird.

13. Verfahren nach Anspruch 12,
wobei als mindestens ein Quantifizierungsparameter ein Schwellenzyklus (Ct-Wert) und/oder ein Quantifizierungszyklus (Cq-Wert) bestimmt wird.

14. Verfahren nach mindestens einem der Ansprüche 1-13,
wobei der Startpunkt der Amplifikation mittels einer Methode eines gleitenden Fensters bestimmt wird, wobei aus allen Messpunkten (MP) eine definierte Anzahl von hintereinander ermittelten Messpunkten (MP) herausgenommen und als Fenster definiert wird, wobei ein spezifischer Parameter in jeweils der ersten und zweiten Hälfte des Fensters bestimmt wird, wobei anschließend das Fenster um einen Messpunkt (MP) verschoben und erneut ein spezifischer Parameter in jeweils der ersten und zweiten Hälfte des Fensters bestimmt wird, wobei das Fenster nach Bestimmung der spezifischen Parameter um jeweils einen Messpunkt (MP) verschoben wird, bis alle möglichen Fenster betrachtet und alle jeweiligen spezifischen Parameter bestimmt wurden, wobei als Startpunkt derjenige Messpunkt (MP) ausgewählt wird, bei dem der Unterschied der spezifischen Parameter aus der ersten und der zweiten Hälfte des Fensters am größten ist.

15. Verfahren nach Anspruch 14,
wobei als spezifischer Parameter ein Mittelwert der Messpunkte (MP) oder ein Maximalwert der Messpunkte (MP) oder ein Anstieg einer Gerade (G1, G2), welche zwischen einem ersten Messpunkt des Fensters und einem mittleren Messpunkt des Fensters sowie zwischen dem mittleren Messpunkt des Fensters und einem letzten Messpunkt des Fensters gebildet wird, berechnet wird.
